# EUROPEAN PATENT SPECIFICATION

(11) **EP 4 503 980 B1**
(45) Date of publication and mention of the grant of the patent: **03.06.2026**
(21) Application number: 23725944.5
(22) Date of filing: 04.01.2023
(51) Int. Cl.: A24F 40/40, A24F 42/20, A24F 40/48, A24F 40/485, A24B 15/16, A61M 11/00, A61M 15/00, A61M 15/06, A24F 40/10, A61M 11/04

(54) **INHALER**
INHALATOR
INHALATEUR

(30) Priority: 24.01.2022 KR 20220009933
(43) Date of publication of application: 12.02.2025
(73) Proprietor: KT & G Corporation, Daejeon 34337 (KR)
(72) Inventor: HONG, Jun Ki, Goyang-si, Gyeonggi-do 10469 (KR); KIM, Do Hoon, Daejeon 34337 (KR); KIM, Moonwon, Anyang-si, Gyeonggi-do 14099 (KR); DO, Eunyeong, Daejeon 34185 (KR); SONG, Hyeyeong, Yongin-si, Gyeonggi-do 17051 (KR)
(74) Representative: AWA Sweden AB
(86) International application number: PCT/KR2023/000121
(87) International publication number: WO 2023/140538

(56) References cited:
- WO-A1-2019/162367
- WO-A1-2020/115155
- WO-A1-2020/245338
- US-A- 4 240 418
- US-A1- 2009 050 137
- US-A1- 2014 144 429
- US-A1- 2017 360 093
- US-A1- 2019 289 909
- US-A1- 2021 361 896
- US-B2- 9 420 829

## Description

### Technical Field

The following description relates to an inhaler.

### Background Art

Research on an inhaler that transfers a target material directly to the lungs of a user has been conducted.

For example, KR20160065204A discloses a dry powder inhaler. Among existing prior art documents, US2009/050137A1, WO2019/162367A1, US2021/361896A1 and US2014/144429A1 may also be mentioned.

In particular, US2009/050137A1 describes an inhaler comprising the features mentioned in the preamble of the present claim 1 and US2021/361896A1 describes an inhaler comprising the features mentioned in the preamble of the present claim 6.

### Disclosure of Invention

### Technical Goals

An inhaler needs to provide a sense of smoking at the time of inhalation of a functional material and thereby increase user satisfaction.

An inhaler needs to control transfer of a functional material regardless of location and environment.

An inhaler needs to induce a phase change of a functional material at a lower temperature to save energy and reduce the size of a battery.

### Technical Solutions

In order to address the above objectives, the present invention provides inhalers as defined in the present claims 1 and 6.

According to embodiments, an inhaler configured to inhale a functional material includes a housing having a mouthpiece, an airflow inlet provided on an outer side of the housing to be separated from the mouthpiece, an airflow channel extending from the airflow inlet to the mouthpiece and having a venturi area of which a diameter of at least a portion thereof decreases and then expands again, a functional material cartridge disposed in the housing and accommodating therein the functional material, and a functional material supply port communicating with the functional material cartridge and the venturi area, in which the functional material may be supplied to the venturi area through the functional material supply port to be transferred to the mouthpiece.

According to the invention, as defined in claim 1, the inhaler includes a heating unit disposed on at least a portion of a perimeter of the venturi area, through which the functional material may be heated.

According to an embodiment, the inhaler may further include an aerosol cartridge disposed in the housing and accommodating therein an aerosol generating material, and an aerosol supply port communicating with the aerosol cartridge and the venturi area, in which an aerosol from the aerosol cartridge may be supplied to the venturi area through the aerosol supply port to be transferred to the mouthpiece.

According to an embodiment, the functional material may include at least one of theanine, caffeine, taurine, nicotine, or a mixture thereof, and the functional material may have a form of fine granules or dry powder.

According to an embodiment, the inhaler may further include a controller configured to open and close the functional material supply port or the aerosol supply port, in which the controller may include a functional material supply mode of opening only the functional material supply port, and a simultaneous supply mode of opening the functional material supply port and the aerosol supply port.

According to other embodiments, an inhaler configured to inhale a functional material includes a housing having a mouthpiece, an airflow inlet provided on an outer side of the housing to be separated from the mouthpiece, an airflow channel extending from the airflow inlet to the mouthpiece and having a venturi area of which a diameter of at least a portion thereof decreases and then expands again, a functional material cartridge communicating with the venturi area and accommodating therein the functional material, and an aerosol cartridge communicating with the venturi area and accommodating therein an aerosol generating material, in which the functional material or aerosol may be supplied to the venturi area to be transferred to the mouthpiece

According to an embodiment, the inhaler may further include a controller configured to selectively supply the functional material or the aerosol to the venturi area, and the controller may include a functional material supply mode of supplying only the functional material to the venturi area, and a simultaneous supply mode of simultaneously supplying the functional material and the aerosol to the venturi area.

According to the invention as defined in claim 6, the inhaler includes a heating unit disposed on at least a portion of a perimeter of the venturi area, through which the functional material or the aerosol generating material may be heated.

### Effects

According to embodiments, it is possible to increase user satisfaction by providing a sense of smoking at the time of inhalation of a functional material.

According to embodiments, it is possible to enable a functional material transfer regardless of location and environment as a functional material transfer mode is configured as two modes.

According to embodiments, it is possible to induce a phase change of a functional material at a lower temperature, thereby saving energy and reducing the size of a battery.

The effects of the inhaler according to embodiments are not limited to the foregoing effects, and other effects that are not described above may be clearly understood from the following description by those having ordinary skill in the art.

### Brief Description of Drawings

The foregoing and other aspects, features, and advantages of certain embodiments of the present disclosure will become apparent from the following detailed description, taken in conjunction with the accompanying drawings.
FIG. 1 is a perspective view of an inhaler according to an embodiment.
FIG. 2 shows an internal structure of an inhaler according to an embodiment.
FIG. 3 shows a state in which an inhaler operates in a functional material supply mode according to an embodiment.
FIG. 4 shows a state in which an inhaler operates in a simultaneous supply mode according to an embodiment.

### Best Mode for Carrying Out Invention

The terms used in the embodiments are selected from among common terms that are currently widely used, in consideration of their functions in the embodiments. However, the terms may be differently construed according to an intention of those having ordinary skill in the art, precedents, or the advent of new technology. Also, in some particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that, when a certain part "includes" a certain component, the part does not exclude another component but may further include another component, unless the context clearly dictates otherwise. Also, terms such as "unit," "module," or the like as used in the disclosure may refer to a part for processing at least one function or operation and may be implemented as hardware, software, or a combination of hardware and software.

As used herein, an expression such as "at least one of" that precedes listed components modifies not each of the listed components but all the components. For example, the expression "at least one of a, b, or c" should be construed as including a, b, c, a and b, a and c, b and c, or a, b, and c.

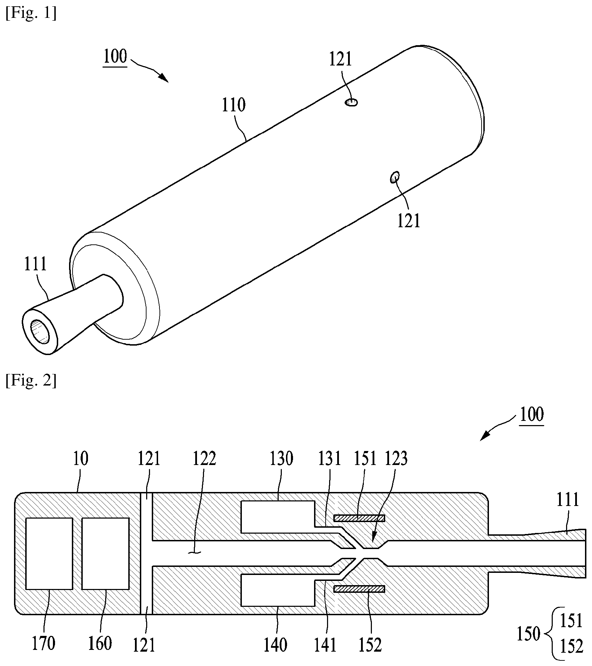

FIG. 1 is a perspective view of an inhaler 100 according to an embodiment, and FIG. 2 shows an internal structure of the inhaler 100 according to an embodiment.

Referring to FIGS. 1 and 2, the inhaler 100 according to an embodiment may include a housing 110, an airflow inlet 121, an airflow channel 122, a functional material cartridge 130, and an aerosol cartridge 140.

In an embodiment, the housing 110 may accommodate components therein and may have an overall elongated tube shape. The housing 110 may be formed of a material such as metal, plastic, or wood.

In an embodiment, the housing 110 may include a first housing part and second housing part that are separable. After the first housing part and the second housing part are separated, the internal components (e.g., the functional material cartridge 130 and the aerosol cartridge 140) may be replaced, and the first housing part and the second housing part may then be combined after the replacement of the internal components.

In an embodiment, a mouthpiece 111 may be provided at one end of the housing 110, and the mouthpiece 111 may include a tube shape having an opening suitable for allowing air and fine particles to pass therethrough. The mouthpiece 111 may be formed to have an outline suitable for the mouth of a user. For example, the mouthpiece 111 may be detachably coupled to the housing 110 by an interference fit or screw coupling method. After the mouthpiece 111 is detached, the internal components (e.g., the functional material cartridge 130 and the aerosol cartridge 140) in the housing 110 may be readily replaced, and the detached mouthpiece 111 may be readily cleaned.

In an embodiment, the airflow inlet 121 may be provided on an outer side of the housing 110. The airflow inlet 121 may be desirably formed near an end of the housing 110 away from the mouthpiece 111, and a plurality of airflow inlets may be provided.

In an embodiment, the airflow channel 122 may be formed to extend from the airflow inlet 121 to the mouthpiece 111 inside the housing 110. For example, as shown in FIG. 2, the airflow channel 122 may be formed to be substantially parallel to a longitudinal direction of the housing 110. Accordingly, during the inhalation by the user, an airflow (e.g., air) introduced into the airflow inlet 121 may pass through the airflow channel 122 and reach the mouth of the user via the mouthpiece 111.

In an embodiment, the airflow channel 122 may include a venturi area 123 having a Venturi tube shape where a diameter decreases and then expands again along the longitudinal direction (i.e., extending direction of the airflow channel 122) of the housing 110. In other words, the venturi area 123 has a structure that causes Venturi effect (i.e., the reduction in fluid pressure that results when a fluid flows through a constricted section of a tube). The venturi area 123 may include a front area having a funnel shape in which a diameter becomes narrower toward the mouthpiece 111 from a front end of the housing 110 (e.g., an end opposite to the rear end where the mouthpiece 111 is provided), a middle area that has a reduced channel diameter and is connected to the front area, and a rear area that has a diameter increasing toward the mouthpiece 111 and is connected to the middle area. During the inhalation by the user, an airflow introduced into the airflow channel 122 through the airflow inlet 121 may travel with a first pressure and a first speed until it reaches the front area of the venturi area 123. After entering the middle area via the front area of the venturi area 123, the airflow may have a second pressure and a second speed. In this case, the first pressure may be greater than the second pressure, and the first speed may be less than the second speed. Subsequently, when passing through the rear area of the venturi area 123 toward the mouthpiece 111, the airflow may have a third pressure and a third speed. In this case, the first pressure and the third pressure may be the same or similar, and the first speed and the third speed may be the same or similar. In the middle area of the venturi area 123, a lowest pressure, compared to those in other areas of the airflow channel 122, may be formed, where a vapor pressure required for a material to change in phase may be lowest compared to those in the other areas of the airflow channel 122.

In an embodiment, the functional material cartridge 130 may be disposed near the venturi area 123. A functional material P may be accommodated in the functional material cartridge 130. The term "functional material" refers to any type of specially designed material with a determined function. For example, the functional material P may serve to improve the taste of an aerosol. The functional material P may include, for example, at least one of theanine, caffeine, taurine, and nicotine. The functional material P may be in the form of fine granules or dry powder.

In an embodiment, a functional material supply port 131 which connects the functional material cartridge 130 and the venturi area 123 may be provided. The functional material supply port 131 may be provided in a tube shape, and one end thereof may be connected to the functional material cartridge 130 and the other end thereof may be connected to the venturi area 123, such that the functional material P in the functional material cartridge 130 may be supplied to the venturi area 123. The other end of the functional material supply port 131 may be desirably connected to the middle area of the venturi area 123.

In an embodiment, a blocking element (not shown) configured to selectively block a functional material supply passage in the functional material supply port 131 may be provided at the functional material supply port 131. The blocking element may block or open the functional material supply passage based on a control signal from a controller 160. The functional material P may thereby be selectively supplied to the airflow channel 122.

According to a modified example, the functional material supply port 131 may be provided as a valve that opens and closes an opening formed on one side of the functional material cartridge 130. In this example, the functional material cartridge 130 may be disposed in contact with the middle area of the venturi area 123. Also, the opening may be configured to communicate with the middle area of the venturi area 123, and the valve may be configured to selectively open or close the opening by a control signal from the controller 160.

**In** an embodiment, the aerosol cartridge 140 may be disposed near the venturi area 123. **In** the aerosol cartridge 140, an aerosol generating material capable of generating an aerosol may be accommodated, and the aerosol generating material may include, for example, vegetable glycerin (VG), propylene glycol (PG), or a mixture thereof.

**In** an embodiment, an aerosol supply port 141 configured to connect the aerosol cartridge 140 and the venturi area 123 may be provided. The aerosol supply port 141 may have a tube shape. Also, one end of the aerosol supply port 141 may be connected to the aerosol cartridge 140 and the other end may be connected to the venturi area 123, such that the aerosol generating material in the aerosol cartridge 140 may be supplied to the venturi area 123. The other end of the aerosol supply port 141 may be desirably connected to the middle area of the venturi area 123.

**In** an embodiment, a blocking element (not shown) configured to selectively block an aerosol generating material supply passage in the aerosol supply port 141 may be provided at the aerosol supply port 141. The blocking element may block or open the aerosol generating material supply passage based on a control signal from the controller 160. The aerosol generating material may thereby be selectively supplied to the airflow channel 122.

According to a modified example, the aerosol supply port 141 may be provided as a valve that opens and closes an opening formed on one side of the aerosol cartridge 140. **In** this example, the aerosol cartridge 140 may be disposed in contact with the middle area of the venturi area 123. Also, the opening may be configured to communicate with the middle area of the venturi area 123, and the valve may be configured to selectively open and close the opening by a control signal from the controller 160.

The inhaler 100 according to an embodiment may further include a heating unit 150 disposed on a perimeter of the venturi area 123. The heating unit 150 may be disposed within the housing 110 to heat the functional material P or the aerosol generating material.

In an embodiment, the heating unit 150 may include a first heater 151 and a second heater 152.

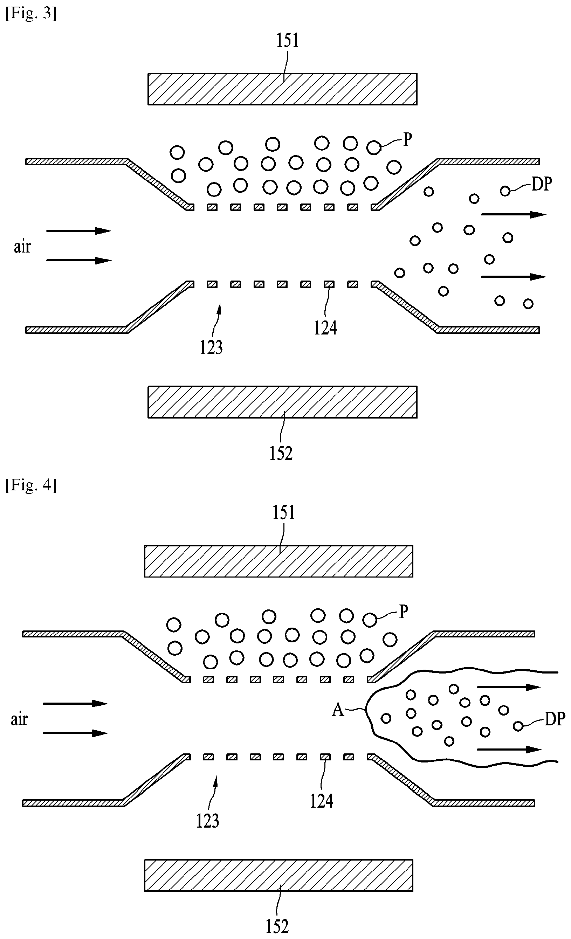

In an embodiment, the first heater 151 may be disposed near the functional material cartridge 130 or the functional material supply port 131 to heat the functional material P supplied to the venturi area 123. The first heater 151 may be, for example, a metal heating wire, a metal heating plate, a ceramic heater, or the like, but is not limited thereto. The first heater 151 may selectively heat the functional material P according to a heating signal from the controller 160. Transfer of the functional material P heated by the first heater 151 will be described in detail below with reference to FIGS. 3 and 4.

In an embodiment, the second heater 152 may be disposed near the aerosol cartridge 140 or the aerosol cartridge supply port 141 to heat the aerosol generating material supplied to the venturi area 123. The second heater 152 may selectively heat the aerosol generating material according to a heating signal from the controller 160. In an embodiment, a liquid transfer means may be provided to transfer the aerosol generating material of the aerosol cartridge 140 to the second heater 152. The liquid transfer means may be, for example, a wick such as cotton fiber, ceramic fiber, glass fiber, or porous ceramic, but is not limited thereto. The second heater 152 may be, for example, a metal heating wire, a metal heating plate, a ceramic heater, or the like, but is not limited thereto. In addition, the second heater 152 may be formed with a conductive filament such as a nichrome wire and may be wound around the liquid transfer means. The second heater 152 may be heated by a current supply from a battery 170, and may heat the aerosol generating material by transferring heat to the aerosol generating material in contact with the second heater 152. As a result, an aerosol A may be generated.

Alternatively, the second heater 152 may be formed with a vibrator (e.g., an ultrasonic element) instead of a heating element. For example, as a voltage (e.g., an alternating voltage) is applied to the vibrator, heat and/or ultrasonic vibration may be generated from the vibrator, and the heat and/or ultrasonic vibration generated from the vibrator may generate an aerosol. For example, by the heat and vibration generated from the vibrator, the viscosity of the aerosol generating material may decrease, allowing the material to become fine particles, and the aerosol A may thereby be generated. Such ultrasonic aerosolization may require less power consumption compared to a heating method, and thus the size of the battery 170 and the apparatus may be reduced. Transfer of the aerosol generating material heated by the second heater 152 will be described in detail below with reference to FIG. 4.

The inhaler 100 according to an embodiment may further include the controller 160 and the battery 170.

In an embodiment, the battery 170 may supply power used to operate the inhaler 100. For example, the battery 170 may supply power to allow the heating unit 150 to be heated and supply power required for the controller 160 to operate. The battery 170 may also supply power required for a display, a sensor, a motor, or the like installed in the inhaler 100 to operate.

In an embodiment, the controller 160 may control the overall operation of the inhaler 100. Specifically, the controller 160 may control operations of the battery 170, the functional material supply port 131, the aerosol supply port 141, and other components included in the inhaler 100. In addition, the controller 160 may verify a state of each of the components of the inhaler 100 and determine whether the inhaler 100 is in an operable state. The controller 160 may include at least one processor. The processor may be implemented as an array of a plurality of logic gates or may be implemented as a combination of a microprocessor and a memory in which a program executable by the microprocessor is stored.

The inhaler 100 according to an embodiment may further include other components in addition to the battery 170 and the controller 160. For example, the inhaler 100 may include a display configured to output visual information and/or a motor configured to output tactile information. In addition, the inhaler 100 may include at least one sensor (e.g., a puff sensor, a temperature sensor, a cartridge insertion detection sensor, etc.).

FIG. 3 shows a state in which the inhaler 100 operates in a functional material supply mode according to an embodiment, and FIG. 4 shows a state in which the inhaler 100 operates in a simultaneous supply mode according to an embodiment.

In an embodiment, the operation mode of the controller 160 may include a functional material supply mode of opening only the functional material supply port 131 and a simultaneous supply mode of opening both the functional material supply port 131 and the aerosol supply port 141.

Referring to FIG. 3, when a user's inhalation is detected while the inhaler 100 according to an embodiment is operating in the functional material supply mode, the controller 160 may transfer a heating signal to the first heater 151 and transfer an open signal to the functional material supply port 131.

In an embodiment, a mesh 124 may be provided outside the middle area of the venturi area 123, and a functional material P in the form of fine granules or dry powder may be introduced into the middle area of the venturi area 123 through the mesh 124. In this case, a pressure lower than atmospheric pressure may be formed in the middle area of the venturi area 123, and thus the vapor pressure of the functional material P may become lower than that at atmospheric pressure. Accordingly, the first heater 151 may induce a phase change of the functional material P at a lower heating temperature than at atmospheric pressure. A functional material DP of which a phase is changed as such may be effectively transferred to the user through the mouthpiece 111.

In another embodiment, when the functional material P is a fine granule larger than the size of a hole of the mesh 124, the functional material P may not be introduced into the venturi area 123. Even in this case, a pressure lower than atmospheric pressure may be formed in the middle area of the venturi area 123, and the vapor pressure of the functional material P may become lower than that at atmospheric pressure. Accordingly, the first heater 151 may induce a phase change of the functional material P at a lower heating temperature than at atmospheric pressure. As a result, the functional material P may transition to a different phase DP and may pass through the mesh 124. For example, the functional material P may transition from a solid to a gas, but embodiments are not limited thereto. Accordingly, the functional material DP may be effectively transferred to the user through the mouthpiece 111.

Referring to FIG. 4, when a user's inhalation is detected while the inhaler 100 according to an embodiment is operating in the simultaneous supply mode, the controller 160 may transfer a heating signal to the first heater 151 and the second heater 152 and transfer an open signal to the functional material supply port 131 and the aerosol supply port 141.

In an embodiment, the mesh 124 may be provided outside the middle area of the venturi area 123, and an aerosol A may be introduced into the middle area of the venturi area 123 through the mesh 124. In this case, a pressure lower than atmospheric pressure may be formed in the middle area of the venturi area 123, and the vapor pressure of an aerosol generating material may become lower than that at atmospheric pressure. Accordingly, the second heater 152 may induce a phase change of the aerosol generating material into the aerosol A at a lower heating temperature than that at atmospheric pressure. As described above, a phase change of a functional material P may also be induced at a low heating temperature of the first heater 151. Such a phase-changed functional material DP and aerosol A may be effectively transferred to the user through the mouthpiece 111. In such a simultaneous operation mode, along with a transfer of the phase-changed functional material DP, a transfer of the aerosol A may be performed, and the user's satisfaction may thereby be maximized.

## Claims

1. An inhaler (100) comprising:
a housing (110) having a mouthpiece (111);
an airflow inlet (121) provided on an outer side of the housing (110) and separated from the mouthpiece (111);
an airflow channel (122) extending in a longitudinal direction from the airflow inlet (121) to the mouthpiece (111) and having a venturi area (123) where a diameter decreases and then expands along the longitudinal direction;
a functional material cartridge (130) disposed in the housing (110) and configured to accommodate a functional material P; and
a functional material supply port (131) communicating with the functional material cartridge (130) and the venturi area (123),
wherein the functional material P is supplied to the venturi area (123) through the functional material supply port (131) and transferred to the mouthpiece (111)
***characterized in that***
a heating unit (150) is disposed on at least a portion of a perimeter of the venturi area (123),
wherein the heating unit (150) includes a first heater (151) disposed near the functional material supply port (131) to heat the functional material P supplied to the venturi area (123).

2. The inhaler (100) of claim 1, further comprising:
an aerosol cartridge (140) disposed in the housing (110) and configured to accommodate an aerosol generating material; and
an aerosol supply port (141) communicating with the aerosol cartridge (140) and the venturi area (123),
wherein an aerosol generating material from the aerosol cartridge (140) is supplied to the venturi area (123) through the aerosol supply port (141) and transferred to the mouthpiece (111).

3. The inhaler (100) of claim 1, wherein the functional material comprises at least one of theanine, caffeine, taurine, and nicotine.

4. The inhaler (100) of claim 3, wherein the functional material has a form of fine granules or dry powder.

5. The inhaler (100) of claim 2, further comprising:
a controller (160) configured to open and close the functional material supply port (131) or the aerosol supply port (141),
wherein the controller (160) opens the functional material supply port (131) and closes the aerosol supply port (141) in a functional material supply mode, and opens the functional material supply port (131) and the aerosol supply port (141) in a simultaneous supply mode.

6. An inhaler (100) comprising:
a housing (110) having a mouthpiece (111);
an airflow inlet (121) provided on an outer side of the housing (110) and separated from the mouthpiece (111);
an airflow channel (122) extending in a longitudinal direction from the airflow inlet (121) to the mouthpiece (111) and having a venturi area (123) where a diameter of at least a portion thereof decreases and then expands along the longitudinal direction;
a functional material cartridge (130) communicating with the venturi area (123) and configured to accommodate a functional material P; and
an aerosol cartridge (140) communicating with the venturi area (123) and configured to accommodate an aerosol generating material,
wherein the functional material P or aerosol generating material is supplied to the venturi area (123) to be transferred to the mouthpiece (111)
***characterized in that***
a heating unit (150) is disposed on at least a portion of a perimeter of the venturi area (123),
wherein the heating unit (150) includes a first heater (151) disposed near the functional material cartridge (130) to heat the functional material P supplied to the venturi area (123).

7. The inhaler (100) of claim 6, further comprising:
a controller (160) configured to selectively supply the functional material or the aerosol generating material to the venturi area (123),
wherein the controller opens a functional material supply (131) and closes an aerosol supply port (141) in a functional material supply mode, and opens the functional material supply port (131) and the aerosol supply port (141) in a simultaneous supply mode.

8. The inhaler (100) of claim 7,
wherein the functional material or the aerosol generating material supplied to the venturi area (123) is heated through the heating unit. (150).

## Patentansprüche

1. Inhalator (100), umfassend:
ein Gehäuse (110), das ein Mundstück (111) aufweist;
einen Luftstromeinlass (121), das an einer Außenseite des Gehäuses (110) und getrennt vom Mundstück (111) vorgesehen ist;
einen Luftstromkanal (122), der sich in einer Längsrichtung von dem Luftstromeinlass (121) bis zum Mundstück (111) erstreckt und einen Venturi-Bereich (123) aufweist, in dem sich ein Durchmesser verringert und dann entlang der Längsrichtung ausdehnt;
eine Funktionsmaterialkartusche (130), die im Gehäuse (110) angeordnet und dazu konfiguriert ist, ein Funktionsmaterial P aufzunehmen; und
eine Funktionsmaterial-Zufuhröffnung (131), die mit der Funktionsmaterialkartusche (130) und dem Venturi-Bereich (123) in Verbindung steht,
wobei das Funktionsmaterial P dem Venturi-Bereich (123) durch die Funktionsmaterial-Zufuhröffnung (131) zugeführt und zum Mundstück (111) übertragen wird,
**dadurch gekennzeichnet, dass**
eine Heizeinheit (150) an mindestens einem Abschnitt eines Rands des Venturi-Bereichs (123) angeordnet ist,
wobei die Heizeinheit (150) ein erstes Heizelement (151) enthält, das in der Nähe der Funktionsmaterial-Zuführöffnung (131) angeordnet ist, um das dem Venturi-Bereich (123) zugeführte Funktionsmaterial P zu erhitzen.

2. Inhalator (100) nach Anspruch 1, ferner umfassend:
eine Aerosolkartusche (140), die im Gehäuse (110) angeordnet und dazu konfiguriert ist, ein Aerosol erzeugendes Material aufzunehmen; und
eine Aerosol-Zufuhröffnung (141), die mit der Aerosolkartusche (140) und dem Venturi-Bereich (123) in Verbindung steht,
wobei dem Venturi-Bereich (123) ein Aerosol erzeugendes Material aus der Aerosolkartusche (140) durch die Aerosol-Zufuhröffnung (141) zugeführt und zum Mundstück (111) übertragen wird.

3. Inhalator (100) nach Anspruch 1, wobei das Funktionsmaterial mindestens eines von Theanin, Koffein, Taurin und Nikotin umfasst.

4. Inhalator (100) nach Anspruch 3, wobei das Funktionsmaterial in Form von feinem Granulat oder trockenem Pulver vorliegt.

5. Inhalator (100) nach Anspruch 2, ferner umfassend:
eine Steuerung (160), die dazu konfiguriert ist, die Funktionsmaterial-Zufuhröffnung (131) oder die Aerosol-Zufuhröffnung (141) zu öffnen und schließen,
wobei die Steuerung (160) in einem Funktionsmaterial-Zufuhrmodus die Funktionsmaterial-Zufuhröffnung (131) öffnet und die Aerosol-Zufuhröffnung (141) schließt und in einem gleichzeitigen Zufuhrmodus die Funktionsmaterial-Zufuhröffnung (131) und die Aerosol-Zufuhröffnung (141) öffnet.

6. Inhalator (100), umfassend:
ein Gehäuse (110), das ein Mundstück (111) aufweist;
einen Luftstromeinlass (121), das an einer Außenseite des Gehäuses (110) und getrennt vom Mundstück (111) vorgesehen ist;
einen Luftstromkanal (122), der sich in einer Längsrichtung von dem Luftstromeinlass (121) bis zum Mundstück (111) erstreckt und einen Venturi-Bereich (123) aufweist, in dem sich ein Durchmesser verringert und dann entlang der Längsrichtung ausdehnt;
eine Funktionsmaterialkartusche (130), die mit dem Venturi-Bereich (123) in Verbindung steht und dazu konfiguriert ist, ein Funktionsmaterial P aufzunehmen; und
eine Aerosolkartusche (140), die mit dem Venturi-Bereich (123) in Verbindung steht und dazu konfiguriert ist, ein Aerosol erzeugendes Material aufzunehmen,
wobei das Funktionsmaterial P oder das Aerosol erzeugende Material, das zum Mundstück (111) übertragen werden soll, dem Venturi-Bereich (123) zugeführt wird,
**dadurch gekennzeichnet, dass**
eine Heizeinheit (150) an mindestens einem Abschnitt eines Rands des Venturi-Bereichs (123) angeordnet ist,
wobei die Heizeinheit (150) ein erstes Heizelement (151) enthält, das in der Nähe der Funktionsmaterialkartusche (130) angeordnet ist, um das dem Venturi-Bereich (123) zugeführte Funktionsmaterial P zu erhitzen.

7. Inhalator (100) nach Anspruch 6, ferner umfassend:
eine Steuerung (160), die dazu konfiguriert ist, das Funktionsmaterial oder das Aerosol erzeugende Material selektiv dem Venturi-Bereich (123) zuzuführen,
wobei die Steuerung in einem Funktionsmaterial-Zufuhrmodus eine Funktionsmaterial-Zufuhr (131) öffnet und eine Aerosol-Zufuhröffnung (141) schließt und in einem gleichzeitigen Zufuhrmodus die Funktionsmaterial-Zufuhröffnung (131) und die Aerosol-Zufuhröffnung (141) öffnet.

8. Inhalator (100) nach Anspruch 7,
wobei das Funktionsmaterial oder das Aerosol erzeugende Material, das dem Venturi-Bereich (123) zugeführt wird, durch die Heizeinheit (150) erhitzt wird.

## Revendications

1. Inhalateur (100) comprenant :
un boîtier (110) comportant un embout buccal (111) ;
une entrée d'écoulement d'air (121) disposée sur un côté extérieur du boîtier (110) et séparée de l'embout buccal (111) ;
un canal d'écoulement d'air (122) s'étendant dans une direction longitudinale à partir de l'entrée d'écoulement d'air (121) vers l'embout buccal (111) et comportant une zone de venturi (123) dans laquelle un diamètre diminue et augmente ensuite le long de la direction longitudinale ;
une cartouche de matériau fonctionnel (130) disposée dans le boîtier (110) et configurée pour accueillir un matériau fonctionnel P ; et
un orifice d'alimentation en matériau fonctionnel (131) communiquant avec la cartouche de matériau fonctionnel (130) et la zone de venturi (123),
dans lequel le matériau fonctionnel P est alimenté vers la zone de venturi (123) à travers l'orifice d'alimentation en matériau fonctionnel (131) et transféré vers l'embout buccal (111),
**caractérisé en ce que**
une unité de chauffage (150) est disposée sur au moins une partie d'un périmètre de la zone de venturi (123),
dans lequel l'unité de chauffage (150) inclut un premier moyen de chauffage (151) disposé à proximité de l'orifice d'alimentation en matériau fonctionnel (131) pour chauffer le matériau fonctionnel P alimenté vers la zone de venturi (123).

2. Inhalateur (100) selon la revendication 1, comprenant en outre :
une cartouche d'aérosol (140) disposée dans le boîtier (110) et configurée pour accueillir un matériau générateur d'aérosol ; et
un orifice d'alimentation en aérosol (141) communiquant avec la cartouche d'aérosol (140) et la zone de venturi (123),
dans lequel un matériau générateur d'aérosol provenant de la cartouche d'aérosol (140) est alimenté vers la zone de venturi (123) à travers l'orifice d'alimentation en aérosol (141) et transféré vers l'embout buccal (111).

3. Inhalateur (100) selon la revendication 1, dans lequel le matériau fonctionnel comprend au moins l'une parmi de la théine, de la caféine, de la taurine et de la nicotine.

4. Inhalateur (100) selon la revendication 3, dans lequel le matériau fonctionnel se présente sous la forme de fines granules ou de poudre sèche.

5. Inhalateur (100) selon la revendication 2, comprenant en outre :
un dispositif de commande (160) configuré pour ouvrir et fermer l'orifice d'alimentation en matériau fonctionnel (131) ou l'orifice d'alimentation en aérosol (141),
dans lequel le dispositif de commande (160) ouvre l'orifice d'alimentation en matériau fonctionnel (131) et ferme l'orifice d'alimentation en aérosol (141) dans un mode d'alimentation de matériau fonctionnel, et ouvre l'orifice d'alimentation en matériau fonctionnel (131) et l'orifice d'alimentation en aérosol (141) dans un mode d'alimentation simultanée.

6. Inhalateur (100) comprenant :
un boîtier (110) comportant un embout buccal (111) ;
une entrée d'écoulement d'air (121) disposée sur un côté extérieur du boîtier (110) et séparée de l'embout buccal (111) ;
un canal d'écoulement d'air (122) s'étendant dans une direction longitudinale à partir de l'entrée d'écoulement d'air (121) vers l'embout buccal (111) et comportant une zone de venturi (123) dans laquelle un diamètre diminue et augmente ensuite le long de la direction longitudinale ;
une cartouche de matériau fonctionnel (130) communiquant avec la zone de venturi (123) et configurée pour accueillir un matériau fonctionnel P ; et
une cartouche d'aérosol (140) communiquant avec la zone de venturi (123) et configurée pour accueillir un matériau générateur d'aérosol,
dans lequel le matériau fonctionnel P ou le matériau générateur d'aérosol est alimenté vers la zone de venturi (123) pour être transféré vers l'embout buccal (111),
**caractérisé en ce que**
une unité de chauffage (150) est disposée sur au moins une partie d'un périmètre de la zone de venturi (123),
dans lequel l'unité de chauffage (150) inclut un premier moyen de chauffage (151) disposé à proximité de la cartouche de matériau fonctionnel (130) pour chauffer le matériau fonctionnel P alimenté vers la zone de venturi (123).

7. Inhalateur (100) selon la revendication 6, comprenant en outre :
un dispositif de commande (160) configuré pour alimenter sélectivement le matériau fonctionnel ou le matériau générateur d'aérosol vers la zone de venturi (123),
dans lequel le dispositif de commande ouvre une alimentation en matériau fonctionnel (131) et ferme un orifice d'alimentation en aérosol (141) dans un mode d'alimentation de matériau fonctionnel, et ouvre l'orifice d'alimentation en matériau fonctionnel (131) et l'orifice d'alimentation en aérosol (141) dans un mode d'alimentation simultanée.

8. Inhalateur (100) selon la revendication 7,
dans lequel le matériau fonctionnel ou le matériau générateur d'aérosol alimenté vers la zone de venturi (123) est chauffé par l'unité de chauffage (150).
